(19) Europäisches Patentamt
European Patent Office
Office européen des brevets

(11) EP 0 583 460 B1

(12) **FASCICULE DE BREVET EUROPEEN**

(45) Date de publication et mention de la délivrance du brevet:
**21.08.1996 Bulletin 1996/34**

(21) Numéro de dépôt: **93905455.7**

(22) Date de dépôt: **05.03.1993**

(51) Int. Cl.[6]: **A61K 7/48**, A61K 7/035

(86) Numéro de dépôt international:
**PCT/FR93/00221**

(87) Numéro de publication internationale:
**WO 93/17660 (16.09.1993 Gazette 1993/22)**

# (54) COMPOSITION COSMETIQUE SOUS FORME DE POUDRE CONTENANT UN LIANT GRAS SILICONE

KOSMETISCHE PUDER-ZUSAMMENSETZUNG ENTHALTEND EIN FETTIGES SILICON BINDEMITTEL

POWDERED COSMETIC COMPOSITION CONTAINING A FATTY SILICONE BINDER

(84) Etats contractants désignés:
**CH DE ES FR GB IT LI**

(30) Priorité: **05.03.1992 FR 9202654**

(43) Date de publication de la demande:
**23.02.1994 Bulletin 1994/08**

(73) Titulaire: **L'OREAL**
**F-75008 Paris (FR)**

(72) Inventeurs:
- **MELLUL, Myriam**
  **F-94240 L'Hay-les-Roses (FR)**
- **LECOMTE, Sophie**
  **F-75013 Paris (FR)**
- **BARA, Isabelle**
  **F-75013 Paris (FR)**
- **DEFOSSEZ, Béatrice**
  **F-75020 Paris (FR)**

(74) Mandataire: **Tonnellier, Jean-Claude et al**
**Cabinet Nony & Cie.**
**29, rue Cambacérès**
**75008 Paris (FR)**

(56) Documents cités:
**EP-A- 0 133 963**
**EP-A- 0 388 582**
**US-A- 5 023 075**

- **PATENT ABSTRACTS OF JAPAN vol. 12, no. 198 (C-502)8 Juin 1986**
- **PATENT ABSTRACTS OF JAPAN vol. 11, no. 3 (C-395)7 Janvier 1987**
- **PATENT ABSTRACTS OF JAPAN vol. 14, no. 57 (C-684)(4000) 2 Février 1990**

EP 0 583 460 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).

## Description

La présente invention a pour objet une composition cosmétique pour la peau, sous forme de poudre contenant un liant gras siliconé.

On sait que certaines compositions cosmétiques telles que des fards à joues, des fards à paupières, des poudres ou fonds de teint pour le visage, sont présentées sous forme de poudres compactées ou coulées. Il s'agit de compositions anhydres appelées "poudres compactes", (ou "compacts") constituées principalement d'un mélange de poudres colorées ou non et d'un liant gras (huiles ou mélange d'huiles et de cires), et mises en forme par compression, ou par coulage dans un conteneur servant de moule. Ces poudres sont généralement utilisées par prélèvement d'une petite quantité de poudre puis application sur la peau, à l'aide d'un applicateur (éponge, houppette ou pinceau).

L'élaboration des agents liants, dans de telles poudres compactes, soulève de nombreuses difficultés. Il faut que le produit final soit suffisamment homogène et compact pour éviter la fragmentation provoquée notamment par les chocs, tout en gardant une bonne aptitude au délitage. Par ailleurs, la composition doit avoir un toucher doux, et être facile à étaler, et ceci de façon uniforme. En outre, le liant doit être compatible avec les pigments, et les spécialistes connaissent les problèmes de dégradation de certains pigments lorsqu'on utilise des liants gras classiques.

On sait par ailleurs que certaines compositions de maquillage sont présentées sous la forme de poudres appelées "poudres libres", dans lesquelles les particules ne sont ni compactées ni dispersées dans une phase continue grasse, mais gardent au contraire leur individualité. De telles poudres libres contiennent souvent un corps gras (huile) qui a pour fonction notamment d'augmenter la douceur d'application, de favoriser l'adhérence de la poudre sur la peau, et de permettre la solubilisation de certains ingrédients actifs. Certaines poudres libres peuvent contenir des quantités relativement élevées d'huile sans que les particules aient tendance à s'agglomérer. C'est le cas notamment des poudres contenant des particules sous forme de microsphères creuses en matériau thermoplastique synthétique ; voir notamment le brevet EP-0 254 612. Mais la formulation de telles poudres libres pose le problème, déjà évoqué ci-dessus, de la dégradation de certains pigments en présence des corps gras utilisés classiquement.

Dans la présente demande, on désigne par l'expression "liant gras" un corps gras ou mélange de corps gras constituant le liant des poudres compactées ou coulées, ainsi qu'un corps gras ou mélange de corps gras présent dans les poudres libres notamment pour augmenter la douceur d'application et favoriser l'adhérence sur la peau.

L'utilisation comme agents liants, dans une poudre compactée, d'huiles de silicone, qui sont des polysiloxanes linéaires (polydiméthylsiloxane ou analogue, en abrégé PDMS) de faible viscosité, en association avec des PDMS de haute viscosité (gommes de silicone), est décrite dans la demande de brevet JP-61-180707.

On a également préconisé l'utilisation comme liants de résines de silicone (produits de polycondensation tridimensionnelle) en association avec des silicones volatiles, dans des poudres cosmétiques ; voir par exemple les demandes de brevet JP-61-065809, JP-61-161211 et JP-62-298512.

Dans la demande de brevet EP-133 963 on a décrit des compositions cosmétiques anhydres contenant des pigments enrobés, dispersés dans un liant à base de PDMS éventuellement en combinaison avec un polysiloxane linéaire substitué (cire de silicone), avec un polysiloxane cyclique et/ou avec une cire classique. Les pigments sont enrobés par liaisons chimiques avec un polysiloxane pour permettre leur dispersion dans le liant.

L'étude de ces divers constituants a montré que les huiles de silicones de faible viscosité, en mélange avec les gommes de silicones, sont intéressantes car elles confèrent notamment à la composition des propriétés de douceur, de facilité d'étalement, et d'homogénéité. Mais les propriétés de tenue du maquillage et de résistance à la chute du compact sont médiocres.

Les cires de polysiloxane permettent d'obtenir une bonne facilité d'étalement et une homogénéité du maquillage acceptable et améliorent les propriétés mécaniques (résistance à la chute). Mais les propriétés de tenue ne sont pas satisfaisantes.

Les résines de silicones confèrent de bonnes propriétés de tenue et de résistance à la chute, mais les compositions manquent de douceur.

Il faut noter que l'association de deux classes de liants siliconés n'apporte généralement pas d'amélioration notable des propriétés.

C'est ainsi que l'addition d'une résine de silicone à des PDMS de haute et basse viscosité diminue la facilité d'étalement, alors que les deux constituants, séparément, donnent des compositions faciles à étaler. De même, l'homogénéité apportée individuellement par les deux constituants se détériore lorsqu'ils sont associés. Par contre, la tenue est améliorée.

L'addition d'une huile de silicone de faible viscosité à une résine de silicone n'améliore pas la douceur et diminue notablement la facilité d'étalement et l'homogénéité.

On a maintenant découvert que l'association de trois classes de silicones (huiles, cires et résines), éventuellement en association avec des gommes de silicones, dans la réalisation du liant gras, permet d'obtenir des poudres dont l'ensemble des propriétés cosmétiques est amélioré ou maintenu à un niveau très satisfaisant. En outre, un tel liant est compatible avec tous les pigments utilisés dans les poudres cosmétiques, y compris les pigments minéraux facilement dégradables tels que le violet de manganèse ou les oxydes de chrome. De plus, le liant conforme à l'invention ne

nécessite pas l'enrobage des pigments pour faciliter leur dispersion.

La présente invention a donc pour objet une composition cosmétique pour la peau, sous forme de poudre anhydre comprenant principalement une phase particulaire solide mélangée à un liant gras contenant un mélange de silicones, caractérisée par le fait que ledit mélange de silicones est constitué par :

(a) au moins une huile de silicone,
(b) au moins une cire de silicone,
(c) au moins une résine de silicone,
(d) éventuellement au moins une gomme de silicone, et
(e) éventuellement au moins une phényldiméthicone,

et que lesdits constituants (a), (b), (c), (d) et (e) sont présents dans le liant respectivement à des concentrations de 12-98,9%, 1-60%, 0,1-25%, 0-3% et 0-20%, en poids par rapport au poids total du mélange de silicones.

La composition selon l'invention peut être une poudre libre, une poudre compacte ou une poudre coulée.

De préférence, les concentrations des constituants du mélange de silicones en poids par rapport au poids total dudit mélange, sont les suivantes :

- gomme de silicone pure : 0-0,4 %.
- cire de silicone : 2-50 %,
- résine de silicone pure : 0,5-15 %,
- phényldiméthicone : 0-15 %,
- huile de silicone : qsp 100 %.

Dans un mode de réalisation particulier, le liant gras est constitué uniquement d'un mélange de silicones tel que défini ci-dessus.

On sait que les huiles de silicone de faible viscosité sont des polysiloxanes linéaires constitués (groupes terminaux exceptés) de motifs de formule (I)

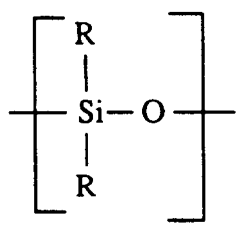

(I)

dans laquelle chaque substituant R représente indépendamment un groupement alkyle inférieur (ayant 1 à 6C).

Le degré de polymérisation (nombre de motifs répétitifs) de ces polysiloxanes de faible viscosité peut aller par exemple de 3 à 2000 environ.

Ces huiles de silicone de faible viscosité peuvent être préparées selon les méthodes connues, ou achetées dans le commerce : par exemple huile Silbione® série 47 (RHONE POULENC), huile série 200 (DOW CORNING), huile SF 96 (GENERAL ELECTRIC).

Les groupes terminaux sont par exemple des groupements triméthylsilyle, diméthyl hydroxyméthylsilyle ou vinyl diméthylsilyle.

Les gommes de silicone utilisables, conformément à la présente invention, sont des polysiloxanes ayant des masses moléculaires élevées, pouvant aller par exemple de 200.000 à 1.000.000. Elles sont utilisées seules ou en mélange dans un solvant. Ce solvant peut être choisi notamment parmi les huiles polydiméthylsiloxanes (PDMS) et les huiles polyphénylméthylsiloxanes (PPMS). Il s'agit également de produits connus et commercialisés, ou pouvant être préparés selon les méthodes connues. On peut citer plus particulièrement les gommes de silicone suivantes : polydiméthylsiloxane/méthylvinylsiloxane, polydiméthylsiloxane/diphénylsiloxane, polydiméthylsiloxane/phénylméthylsiloxane, et polydiméthylsiloxane/diphénylsiloxane/méthylvinylsiloxane. Parmi les gommes de silicone commerciales, on peut citer celles vendues sous les dénomination SE30 (GENERAL ELECTRIC), TP232 (UNION CARBIDE), Q2-1403 (DOW CORNING), ou la série VISCASIL® (GENERAL ELECTRIC).

Les cires de silicone utilisables dans le liant gras de la présente invention sont des polysiloxanes substitués solides ou liquides à température ambiante. Ce sont de préférence des fluides ou solides à bas point de fusion. Il s'agit notamment de polysiloxanes linéaires substitués constitués essentiellement (les groupes terminaux mis à part) de motifs de formules II et III, dans les proportions molaires respectives m et n :

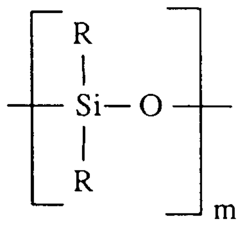

(II)

et

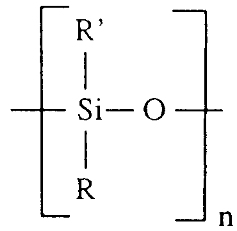

(III)

dans lesquelles chaque substituant R est défini comme précédemment,

chaque R' représente indépendamment un alkyle (linéaire ou ramifié) éventuellement insaturé, ayant 6-30 atomes de carbone, ou bien un groupement -X-R",

chaque X représente indépendamment :

-O-,

$-(CH_2)_a-O-CO-$,

$-(CH_2)_b-CO-O-$,

a et b représentent indépendamment des nombres pouvant varier de 0 à 6, et

chaque R" représente indépendamment un groupement alkyle, éventuellement insaturé, ayant 6 à 30 atomes de carbone

m est un nombre pouvant varier de 0 à 400, et en particulier de 0 à 100,

n est un nombre pouvant varier de 1 à 200, et en particulier de 1 à 100,

la somme (m + n) étant inférieure à 400, et en particulier inférieure ou égale à 100.

Ces cires de silicones sont connues ou peuvent être préparées selon les méthodes connues. Parmi les cires de silicones commerciales de ce type, on peut citer notament celles vendues sous les dénominations Abilwax® 9800, 9801 ou 9810 (GOLDSCHMIDT), KF910 et KF7002 (SHIN ETSU), ou 176-1118-3 et 176-11481 (GENERAL ELECTRIC).

Les cires de silicone utilisables peuvent également être choisies parmi les composés de formule IV

$$R_1-Si(CH_3)_2-O-[Si(R)_2-O-]_z-Si(CH_3)_2-R_2 \qquad (IV)$$

dans laquelle R est défini comme précédemment,

$R_1$ représente un groupement alkyle de 1 à 30 C, un groupement alcoxy de 6 à 30 C, ou un groupement de formule :

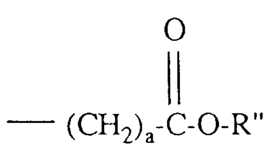

ou

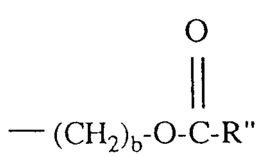

$R_2$ représente un groupement alkyle de 6 à 30 C, un groupement alcoxy ayant de 6 à 30 C ou ou un groupement de formule :

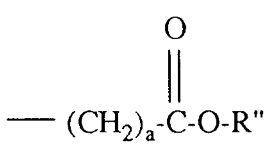

ou

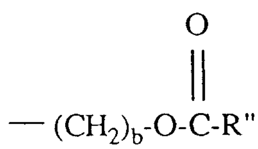

a et b représentant un nombre de 0 à 6,

R" étant un alkyle de $C_6$ à $C_{30}$.

et z est un nombre pouvant varier de 1 à 100.

Parmi les cires de silicone de formule IV, qui sont des produits connus ou pouvant être préparés selon les méthodes connues, on citera notamment les produits commerciaux suivants : Abilwax® 2428, 2434 et 2440 (GOLDSCHMIDT), ou VP 1622 et VP 1621 (WACKER).

Les résines de silicones sont des produits d'hydrolyse et de polycondensation de mélanges de siloxanes de formules $(R)_3SiOCH_3$ et $Si(OCH_3)_4$, R représentant un groupement alkyle ayant de 1 à 6 C.

Ces résines de silicone sont connues ou peuvent être préparées selon des méthodes connues. Parmi les résines de silicone commerciales utilisables, on peut citer par exemple celles qui sont vendues sous les dénominations DC 593 (DOW CORNING) ou SS 4230 (GENERAL ELECTRIC).

Les phényldiméthicones sont des produits connus répondant à la formule VI :

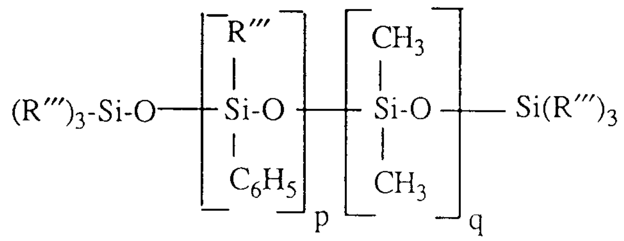

(VI)

dans laquelle

q est un nombre pouvant varier de 0 à 5 000,

p est un nombre pouvant varier de 1 à 5 000,

et chaque R''' représente indépendamment un groupement méthyle, phényle, ou triméthyl silyloxy.

Ces phényldiméthicones peuvent être utilisées comme ingrédients facultatifs, permettant d'améliorer la douceur d'application.

Généralement, le liant gras peut représenter de 0,5 à 25 % en poids, de préférence de 3 à 20 %, par rapport au poids total de la composition.

La phase particulaire de la composition est constitué par des pigments et/ou charges habituellement utilisés dans de telles compositions cosmétiques. Les pigments sont choisis parmi les pigments minéraux et/ou organiques, et/ou les pigments nacrés.

Ces pigments peuvent représenter jusqu'à 70 % du poids de la composition finale.

Parmi les pigments minéraux on peut citer, à titre d'exemple :

- le dioxyde de titane (rutile ou anatase), éventuellement traité en surface, et codifié dans le Color Index sous la référence CI77891 ;
- les oxydes de fer noir, jaune, rouge et brun, codifiés sous les références CI 77499, 77492, 77491;
- le violet de manganèse (CI 77742);
- le violet d'outremer (CI 77007);
- le bleu d'outremer (CI 77007);
- l'oxyde de chrome (CI 77288);
- l'oxyde de chrome hydraté (CI 77289) et
- le bleu ferrique (CI 77510).

Parmi les pigments organiques on peut citer, en particulier, les pigments :

- D & C red n° 3 (CI 45430:1)
- D & C red n° 6 (CI 15850:2)
- D & C red n° 7 (CI 15850;1)
- D & C red n° 9 (CI 15585;1)
- D & C red n° 13 (CI 15630;3)
- D & C red n° 19 (CI 45170)
- D & C red n° 21 (CI 45380:2)
- D & C red n° 27 (CI 45410:1)
- D & C red n° 30 (CI 73360)
- D & C red n° 36 (CI 12085),
- le noir de carbone (CI 77266) et les laques à base de carmin de cochenille (CI 75470).

Les pigments nacrés peuvent être choisis notamment parmi les pigments nacrés blancs, tels que le mica recouvert d'oxyde de titane ou d'oxychlorure de bismuth. On peut utiliser également des pigments nacrés colorés, tel que le mica titane coloré avec des oxydes de fer, le mica titane coloré avec du bleu ferrique ou de l'oxyde de chrome, le mica titane coloré avec un pigment organique du type précité, ainsi que des pigments nacrés à base d'oxychlorure de bismuth.

Les charges sont choisies notamment parmi :

- Le talc, qui est un silicate de magnésium hydraté, utilisé sous forme de particules généralement de dimensions inférieures à 40 µm ; le talc possède des propriétés absorbantes de l'humidité et est utilisé surtout en raison de son toucher onctueux ;
- les micas, qui sont des aluminosilicates de compositions variées, qui se présentent sous la forme d'écailles ayant des dimensions de 2 à 200 µm, de préférence de 5 à 70 µm et une épaisseur de 0.1 à 5 µm, de préférence de 0.2 à 3 µm. Les micas peuvent être d'origine naturelle (par exemple muscovite, margarite, roscoelithe, lipidolithe, biotite) ou d'origine synthétique. Les micas sont généralement transparents et permettent de conférer à la peau un aspect satiné ;
- l'amidon, modifié ou non, en particulier l'amidon de riz ;
- la silice ;
- l'alumine ;
- le nitrure de bore ;
- le kaolin, qui est un silicate d'aluminium hydraté, qui se présente sous la forme de particules de forme isotrope, et qui possède de bonnes propriétés d'absorption des corps gras ;
- les oxydes de zinc et de titane : ces oxydes ont un toucher onctueux, ont un bon pouvoir couvrant et ont une opacité importante ; on peut aussi utiliser les formes nanopigmentaires de ces produits ;
- le carbonate de calcium précipité qui, sous forme de particules de dimensions inférieures à 10 µm environ, a un toucher onctueux et permet d'obtenir un aspect mat ;
- le carbonate ou l'hydrocarbonate de magnésium, qui possèdent notamment des propriétés de fixation des parfums ;
- des savons métalliques dérivés d'acide organique carboxylique ayant de 8 à 22 atomes de carbone, de préférence de 12 à 18 atomes de carbone, par exemple le stéarate de zinc, de magnésium ou de lithium, le laurate de zinc, le myristate de magnésium, etc... Ces savons, présents généralement sous la forme de particules ayant des dimensions inférieures à 10 µm, ont un toucher onctueux et facilitent l'adhérence de la poudre sur la peau ;
- les poudres de polymères (ou copolymères) synthétiques choisis parmi le polyéthylène et ses dérivés (par ex : polytétrafluoroéthylène, polystyrène...), les polyacrylates, les polyméthacrylates, les polyesters ou les polyamides etc..., par exemple la poudre de nylon.
- les poudres sous forme de microsphères creuses en matériau synthétique thermoplastique, dont la partie creuse contient un gaz.

Les microsphères creuses sont préparées selon les procédés connus, tels que ceux décrits dans le brevet FR 3 615 972 ou la demande de brevet européen n° 0 056 219.

Ces microsphères peuvent être réalisées en tous matériaux thermoplastiques non toxiques et non irritants. Ces matériaux peuvent être par exemple des polymères ou copolymères de dérivés éthyléniques (par exemple polyéthylène, polystyrène, copolymère chlorure de vinyle-acrylonitrile, etc.) des polyesters, des polymères urée-formaldéhyde, des copolymères de chlorure de vinylidène (par exemple chlorure de vinylidène-acrylonitrile), etc.

Les charges peuvent représenter jusqu'à 95 % du poids total de la composition de l'invention.

Les pigments et charges peuvent être enrobés, si désiré, par des substances telles que notamment des acides aminés, des silicones, des savons métalliques ou du collagène, notamment afin de modifier leur état de surface. On peut également modifier l'état de surface par greffage chimique ou adsorption de molécules siliconées, d'autres molécules pouvant convenir également, comme le titanate de triisostéaroyle.

Divers additifs usuels peuvent également être introduits dans la composition. Généralement, ces additifs, pris ensemble, ne représentent pas plus de 10%, et notamment pas plus de 5% en poids par rapport au poids total de la composition. La composition de l'invention peut donc contenir au moins un additif choisi notamment parmi des antiseptiques (par exemple trichloro diphényl éther, agents cationiques, acide borique, etc...) qui sont utilisés notamment dans les poudres désodorisantes pour le corps ou les pieds et dans les poudres pour bébés ; des agents astringents, qui sont utilisés dans les poudres désodorisantes ou dans les poudres pour les pieds, tels que l'hydroxychlorure d'aluminium ou les aluns ; des filtres solaires ; des agents cicatrisants ; des agents antiradicaux libres ; des vitamines ; des agents adoucissants ; des agents émollients, notamment des huiles telles que des esters d'acides gras avec un alcool gras en $C_{10}$ à $C_{22}$ ou avec un alcool inférieur (par exemple citrate de triisocétyle, myristate de myristyle, etc.) ou des huiles végétales (notamment huile de jojoba etc.) des huiles minérales (notamment l'huile de vaseline, etc.) ou d'origine animale (notamment lanoline, etc;) ; des agents hydratants (glycérol, sorbitol, etc.); des agents dépigmentants ; des parfums ; des agents de consistance (gommes naturelles ou synthétiques); etc.

Les compositions de l'invention peuvent être présentées notamment sous la forme de fards à joues, de fards à paupières, de poudres pour le maquillage du visage, de poudres corporelles (parfumées et/ou désodorisantes), y compris de poudres pour les pieds, etc....

Les compositions de l'invention peuvent être préparées selon les méthodes usuelles, par exemple selon l'un des procédés suivants :

Procédé 1 : (pour poudres compactées)

Dans un premier temps, on mélange les pigments et/ou les charges, ainsi que les additifs poudreux, puis on ajoute le liant et/ou éventuellement les agents de consistance ainsi que d'autres ingrédients éventuels, et le tout est mélangé et/ou éventuellement broyé.

On pourra éventuellement chauffer le liant si nécessaire.

Le mélange est ensuite compacté à l'aide d'une presse dans des coupelles métalliques.

Procédé 2 : (pour poudres coulées)

Tous les constituants de la formule sont mélangés et mis en suspension dans un solvant (eau, hexane, isopropanol, éthanol, etc...).

La pâte obtenue est alors coulée dans une coupelle, puis le solvant est évaporé.

Procédé 3 : (pour poudres libres)

Les pigments et/ou charges ainsi que les additifs poudreux sont mélangés, puis on ajoute le liant et éventuellement les agents de consistance ainsi que d'autres ingrédients éventuels et le tout est mélangé et éventuellement broyé.

Le cas échéant le liant est chauffé. Si on le souhaite, le mélange peut être tamisé avant un conditionnement dans un récepteur convenable.

L'invention a également pour objet l'utilisation d'un mélange de silicones comme agent liant, dans la préparation d'une composition cosmétique anhydre se présentant sous la forme d'une poudre (libre, compactée ou coulée) à base principalement de particules solides mélangées à un liant gras, ledit mélange de silicones étant tel que défini précédemment. Les compositions obtenues sont appliquées sur la peau selon les méthodes usuelles.

Les exemples suivants illustrent l'invention. Dans ces exemples, les quantités des divers ingrédients sont données en parties en poids.

EXEMPLE 1 : **Fard à joues**

| | |
|---|---|
| Partie A | |
| Dioxyde de titane | 10,00 |
| Mica-titane | 10,00 |
| Stéarate de zinc | 4,00 |
| D & C Red 30 | 0,50 |
| Talc | 69,30 |
| Partie B. | |
| Cétyl diméthicone | 0,08 |
| Béhénoxy diméthicone | 1,60 |
| Triméthylsiloxysilicate à 33 % dans un PDMS basse viscosité | 0,53 |
| Polydiméthylsiloxane haute viscosité à 14% de matière active dans un PDMS basse viscosité | 0,01 |
| Polydiméthylsiloxane basse viscosité | 3,78 |
| Conservateurs | 0,2 |
| | 100,00 |

Origine des produits :

Cétyl diméthicone : Abilwax® 9801 (GOLDSCHMIDT)
Béhénoxy diméthicone : Abilwax® 2440 (GOLDSCHMIDT)
Triméthylsiloxysilicate à 33% dans un PDMS basse viscosité ; DC593 (DOW CORNING).
Polydiméthylsiloxane haute viscosité : Q2-1403 (DOW CORNING) : solution à 14% dans un PDMS basse viscosité.
Polydiméthylsiloxane basse viscosité : PDMS 10 centistockes commercialisé par la Société GOLDSCHMIDT.

*Mode opératoire* :

1) Mélanger les constituants de la phase A.
2) Ajouter la phase B. Mélanger à nouveau.
3) Broyer éventuellement.
4) Tamiser.
5) Compacter dans une coupelle métallique.

Cette formule est très facile à déliter et donne un maquillage très homogène.

EXEMPLE 2 ; **Poudre compactée pour le visage**

| | |
|---|---|
| Partie A. | |
| Séricite. | 65,80 |
| Mica | 15,00 |
| Poudre de polyéthylène | 5,00 |
| Dioxyde de titane | 2,00 |
| Oxydes de fer | 8,00 |
| Partie B. | |
| Cétyl diméthicone | 0,15 |
| Béhénoxy diméthicone | 0,30 |
| Triméthylsiloxysilicate (DC 593) | 0,75 |
| Polydiméthylsiloxane basse viscosité | 2,80 |
| Conservateurs, | 0.2 |
| | 100,00 |

Origine des produits : voir exemple 1
*Mode opératoire* : celui de l'exemple 1
Ce produit présente une grande douceur à l'application.

EXEMPLE 3 : **Fard à paupières**

| | |
|---|---|
| Partie A. | |
| Mica-titane | 40,00 |
| Oxyde de chrome vert | 6,00 |
| Talc, | 43.80 |
| Partie B. | |
| Cétyl diméthicone | 1,24 |
| Triméthylsiloxysilicate (DC 593) | 1,98 |
| Polydiméthylsiloxane basse viscosité | 6,78 |
| Conservateur, | 0.20 |
| | 100,00 |
| C. Ethanol | 85,00 |

Origine des produits : voir exemple 1

*Mode opératoire* :

1) Mélanger les constituants de la phase A.
2) Ajouter la phase B et la phase C et mélanger à nouveau.
3) La pâte obtenue est coulée ou injectée directement dans une coupelle.
4) Le solvant est évaporé.

Ce produit a une bonne facilité d'application et une grande douceur.

EXEMPLE 4 : **Fard à paupières**

| | |
|---|---|
| Partie A. | |
| Mica | 15,00 |
| Poudre de polyamide | 10,00 |
| Oxyde de fer | 10,00 |
| Violet de manganèse | 20,00 |
| Talc, | 38,80 |
| Partie B. | |
| Cétyl diméthicone | 0,21 |
| Béhénoxy diméthicone | 0,44 |
| Triméthylsiloxysilicate (DC 593) | 1,12 |
| Polydiméthylsiloxane basse viscosité | 4,23 |
| Conservateurs | 0,20 |
| | 100,00 |

Origine des produits : voir exemple 1

*Mode opératoire* :

1) Mélanger les constituants de la phase A.
2) Ajouter la phase B et mélanger à nouveau
3) Broyer éventuellement
4) Tamiser
5) Compacter dans une coupelle métallique.

Ce produit est très doux et très facile à étaler.

EXEMPLE 5 : **Poudre libre**

| | |
|---|---|
| Partie A. | |
| Mica | 67,50 |
| Poudre de polyamide | 25,00 |
| Stéarate de zinc | 2,00 |
| Oxydes de fer | 1,00 |
| Parfum (imprégné dans carbonate de magnésium) | 1,5 |
| Partie B. | |
| Abilwax® 9801 | 1,5 |
| DC593 | 0,5 |
| PDMS basse viscosité | 1,0 |
| | 100,00 |

Le PDMS basse viscosité est celui commercialisé sous la dénomination Abil® 10 (GOLDSCHMIDT).

*Mode opératoire* :

1) Mélanger les constituants de la partie A
2) Ajouter la partie B et mélanger à nouveau
3) Broyer et tamiser.

EXEMPLES COMPARATIFS

On a étudié les propriétés de poudres compactées obtenues en utilisant comme agents liants divers silicones ou mélanges de silicones. Dans tous les cas la phase particulaire était la même qu'à l'exemple 4 (A) ci-dessus, la proportion d'agent liant étant de 6 %. Les compositions ont été préparées comme à l'exemple 4.

Les compositions obtenues ont été testées par des utilisatrices qui devaient indiquer, pour la propriété étudiée, si la composition testée donne un résultat moyen (0), un bon résultat (+) ou un très bon résultat (++).

Les propriétés étudiées étaient les suivantes :

a) Propriétés mécaniques du compact.

- résistance à la chute : le test consiste à évaluer la cohésion du produit compacté par mesure de la perte de masse de poudre après 10 chutes normalisées d'une hauteur de 20cm,

b) Propriétés cosmétiques :

- douceur d'application : le test consiste à évaluer (évaluation sensorielle) la douceur à l'application ;
- facilité d'étalement : le test consiste à évaluer la facilité d'étendre la poudre et de la déposer sur toute la surface à maquiller ;
- adhérence : le test consiste à évaluer l'aptitude de la poudre à se déposer et à rester en place sur la peau ;
- tenue : le test consiste à évaluer l'aptitude de la poudre à rester sur la peau au bout de 4 heures ;
- homogénéité : le test consiste à évaluer l'uniformité de la couche de poudre sur la peau après maquillage.

Les compositions étudiées et les résultats sont résumés dans les tableaux 1 et 2. Les compositions étudiées dans le tableau 2 sont des compositions selon l'invention, tandis que les compositions du tableau 1 sont des compositions de comparaison.

TABLEAU 1

| Compositions de comparaison | | | | | | | | | |
|---|---|---|---|---|---|---|---|---|---|
| COMPOSITIONS | 1 | 2 | 3 | 4 | 5 | 6 | 7 | 8 | 9 |
| PDMS BASSE VISCOSITE | 100% | | | | | 60% | 30% | 50% | 50% |
| PDMS HAUTE VISCOSITE * | | 100% | | | | 40% | 20% | | |
| BEHENOXY DIMETHICONE | | | 100% | | | | | 50% | |
| CETYL DIMETHICONE | | | | 100% | | | | | |
| TRIMETHYL-SILOXYSILICATE | | | | | 100% | | 50% | | 50% |
| PROPRIETES | | | | | | | | | |
| Résist. chute | 0 | 0 | ++ | + | ++ | 0 | 0 | ++ | + |
| Douc. à l'applic. | ++ | 0 | + | + | 0 | + | + | ++ | 0 |
| Fac. d'étalement | ++ | + | ++ | + | + | + | + | + | 0 |
| Tenue | + | 0 | 0 | 0 | ++ | 0 | ++ | 0 | ++ |
| Homogénéité | ++ | + | + | ++ | + | ++ | 0 | ++ | 0 |
| Adhérence | 0 | ++ | + | + | + | + | ++ | 0 | + |
| Origine des produits : voir exemple 1 | | | | | | | | | |

* Solution à 14% de matière active dissoute dans un PDMS de basse viscositè

TABLEAU 2

| Compositions n° 10 à 15 selon l'invention | | | | | | |
|---|---|---|---|---|---|---|
| COMPOSITIONS | 10 | 11 | 12 | 13 | 14 | 15 |
| CETYL DIMETHICONE | 3,8 | 1,3 | 12,4 | 20,0 | 20,8 | 2,0 |
| BEHENOXY DIMETHICONE | 7,3 | 26,7 | - | 20,0 | 0,5 | 25,0 |
| TRIMETHYL-SILOXYSILICATE * | 18,7 | 8,8 | 19,8 | 6,6 | 24,7 | 3,0 |
| PDMS HAUTE VISCOSITE** | - | 0,2 | - | 0,1 | - | 1,0 |
| PDMS BASSE VISCOSITE | 70,2 | 63,0 | 67,8 | 53,3 | 54,0 | 69,0 |
| PROPRIETES | | | | | | |
| Résist. chute | + | + | + | ++ | ++ | + |
| Douceur à l'application | ++ | ++ | ++ | + | + | ++ |
| Facilité d'étalement | + | + | ++ | ++ | + | ++ |
| Tenue | + | + | ++ | + | ++ | + |
| Homogénéité | + | ++ | ++ | + | ++ | ++ |
| Adhérence | ++ | + | + | ++ | + | + |
| Origine des produits : voir l'exemple 1 | | | | | | |

* à 33% de matière active dans un PDMS da basse viscositè
** à 14% de matière active dans un PDMS de basse viscositè

2) Résultats de conservation :

On a comparé les compositions n° 10, 11 et 12 précédentes avec une composition contenant un liant classique et la même phase particulaire. Les compositions sont conservées à l'étuve (40°C) pendant 2 mois.

Au bout de ce temps on évalue subjectivement l'intensité de l'odeur, notée de la façon de la suivante :

- très intense : +++
- intense : ++
- assez intense : +
- pas d'odeur : 0

Les compositions classiques contenaient le liant suivant :

COMPOSITION 16 :

- huile de vaseline : 55 %
- alcool oléique : 30 %
- lanoline liquide : 10 %
- huile de ricin : 5 %

COMPOSITION 17 :

- huile de vaseline : 85 %
- vaseline blanche : 15 %

Les résultats sont résumés dans le tableau 3 suivant :

TABLEAU 3

| COMPOSITIONS | 10 | 11 | 12 | 16 | 17 |
|---|---|---|---|---|---|
| Odeur | 0 | 0 | 0 | +++ | + |

3) <u>Réalisation de compositions contenant des pigments difficiles à mettre en oeuvre</u>

On sait que certains pigments, tels que l'oxyde de chrome, le bleu d'outremer, le violet de manganèse, conduisent à des formulations difficiles à mettre en oeuvre et peu homogènes au maquillage.

Les compositions testées étaient les suivantes :

- talc : 40 %
- oxydes de chrome : 15 %
- mica : 20 %
- mica-titane enrobé d'oxyde de chrome : 15 %
- liant : 10 %

L'oxyde de chrome utilisé est un mélange 50/50 d'oxyde de chrome et d'hydroxyde de chrome.

Pour les compositions 18, 19 et 20 étudiées, l'agent liant était le suivant :

- Composition 18 : liant de la composition 16
- Composition 19 : liant de la composition 11 (selon l'invention)
- Composition 20 : liant de la composition 12 (selon l'invention)

Les résultats sont résumés dans le tableau suivant (Tableau 4) :

TABLEAU 4

| COMPOSITIONS TESTEES | 18 | 19 | 20 |
|---|---|---|---|
| Douceur | 0 | + | + |
| Facilité d'étalement | + | ++ | ++ |
| Homogénéité | 0 | + | ++ |

**Revendications**

1. Composition cosmétique pour la peau, sous forme de poudre anhydre comprenant principalement une phase particulaire solide mélangée à un liant gras contenant un mélange de silicones caractérisée par le fait que ledit mélange de silicones, est constitué par :

   (a) au moins une huile de silicone,
   (b) au moins une cire de silicone,
   (c) au moins une résine de silicone,
   (d) éventuellement au moins une gomme de silicone, et
   (e) éventuellement au moins une phényldiméthicone.

   et que lesdits constituants (a), (b), (c), (d) et (e) sont présents dans le liant respectivement à des concentrations de 12-98,9%, 1-60%, 0,1-25%, 0-3% et 0-20%, en poids par rapport au poids total du mélange de silicones.

2. Composition selon la revendication précédente, caractérisée par le fait que les concentrations des constituants du mélange de silicones, en poids par rapport au poids total dudit mélange sont les suivants :

- cire de silicone : 2-50 %,
- résine de silicone : 0,5-15 %,
- gomme de silicone : 0-0,4 %.
- phényldiméthicone : 0-15%,
- huile de silicone : qsp 100 %.

**3.** Composition selon l'une quelconque des revendications précédentes, caractérisée par le fait que ledit liant gras est constitué dudit mélange de silicones.

**4.** Composition selon l'une quelconque des revendications précédentes, caractérisée par le fait que l'huile de silicone de faible viscosité est au moins un polysiloxane linéaire constitué (groupes terminaux exceptés) de motifs de formule I

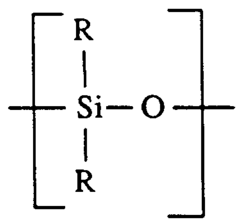

(I)

dans laquelle chaque substituant R représente indépendamment un groupement alkyle inférieur ayant de 1 à 6 C,

ledit polysiloxane ayant un degré de polymérisation de 3 à 2000.

**5.** Composition selon l'une quelconque des revendications précédentes, caractérisée par le fait que la cire de silicone est constituée essentiellement par au moins un polysiloxane linéaire substitué constitué essentiellement (les groupes terminaux mis à part) de motifs de formules (II) et (III), dans les proportions molaires respectives m et n :

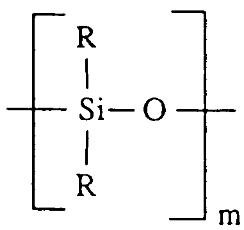

et

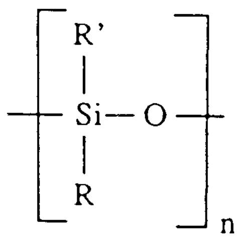

(II) (III)

dans lesquelles chaque substituant R est un groupement alkyle ayant 1 à 6 C,

chaque R' représente indépendamment un alkyle, éventuellement insaturé, ayant 6 à 30 atomes de carbone, ou bien un groupement -X-R",

chaque X représente indépendamment :

-O-,

$-(CH_2)_a-O-CO-$,

$-(CH_2)_b-CO-O-$,

a et b représentent indépendamment des nombres pouvant varier de 0 à 6, et

chaque R" représente indépendamment un groupement alkyle, éventuellement insaturé, ayant 6 à 30 atomes de carbone

m est un nombre pouvant varier de 0 à 400, et en particulier de 0 à 100,

n est un nombre pouvant varier de 1 à 200, et en particulier de 1 à 100,

la somme (m + n) étant inférieure à 400, et en particulier inférieure ou égale à 100.

6. Composition selon l'une quelconque des revendications précédentes, caractérisée par le fait que ladite cire de silicone contient au moins un composé de formule IV :

$$R_1\text{-}Si(CH_3)_2\text{-}O\text{-}[Si(R)_2\text{-}O\text{-}]_z\text{-}Si(CH_3)_2\text{-}R_2 \qquad (IV)$$

dans laquelle chaque R est un groupement alkyle ayant 1 à 6 C,
$R_1$ représente un groupement alkyle ayant de 1 à 30C, un groupement alcoxy ayant de 6 à 30 C, ou un groupement :

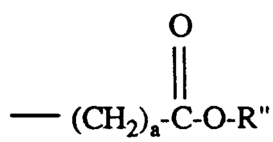
ou
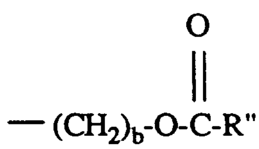

$R_2$ représente un groupement alkyle de 6 à 30 C, un groupement alcoxy ayant de 6 à 30 C ou un groupement :

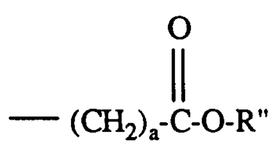
ou
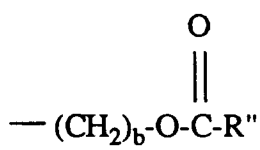

a et b représentant un nombre de 0 à 6,
R" étant un alkyle de $C_6$ à $C_{30}$.
et z est un nombre pouvant varier de 1 à 100.

7. Composition selon l'une quelconque des revendications précédentes, caractérisée par le fait que ladite résine de silicone est constituée par des produits d'hydrolyse et de polycondensation des mélanges de siloxanes de formule $(R)_3SiOCH_3$ et $Si(OCH_3)_4$, R représentant un groupement alkyle ayant de 1 à 6 C.

8. Composition selon l'une quelconque des revendications précédentes, caractérisée par le fait que ladite gomme de silicone est un polysiloxane ayant une masse moléculaire de 200 000 à 1 million.

9. Composition selon l'une quelconque des revendications précédentes, caractérisée par le fait que ladite phényldiméthicone répond à la formule VI.

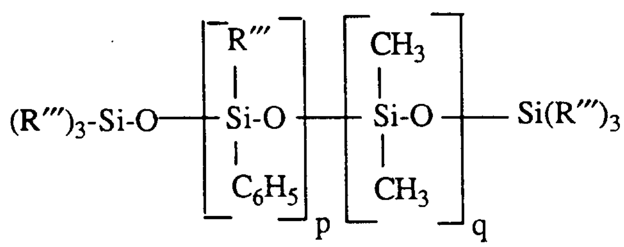
(VI)

dans laquelle
q est un nombre pouvant varier de 0 à 5 000,
p est un nombre pouvant varier de 1 à 5 000,
et chaque R''' représente indépendamment un groupement méthyle, phényle, ou triméthyl silyloxy.

10. Composition selon l'une quelconque des revendications précédentes, caractérisée par le fait que ledit mélange de silicones représente de 0,5 à 25%, et en particulier de 3 à 20% en poids par rapport au poids total de la composition.

11. Composition selon l'une quelconque des revendications précédentes, caractérisée par le fait qu'elle comprend en outre, au moins un agent additif choisi parmi des antiseptiques, des agents astringents, des filtres solaires, des agents cicatrisants, des agents anti-radicaux libres, des vitamines, des agents adoucissants, des agents émollients, des agents dépigmentants, des parfums et des agents de consistance.

12. Composition selon la revendication précédente, caractérisée par le fait que les additifs, pris ensemble, ne représentant pas plus de 10%, et en particulier pas plus de 5%, du poids total de la composition.

13. Composition selon l'une quelconque des revendications précédentes, caractérisée, par le fait qu'elle se présente sous la forme d'un fard à paupières, d'un fard à joues, d'une poudre pour le maquillage du visage, ou d'une poudre corporelle.

14. Utilisation d'un mélange de silicones dans la préparation d'une composition cosmétique anhydre se présentant sous la forme d'une poudre constituée de particules solides mélangées à un liant gras comprenant un mélange de silicones, caracterisée par le fait que ledit mélange de silicones est constitué par :

    a) au moins une huile de silicone,
    (b) au moins une cire de silicone,
    (c) au moins une résine de silicone,
    (d) éventuellement au moins une gomme de silicone, et
    (e) éventuellement au moins une phényldiméthicone,

    lesdits constituants (a), (b), (c), (d) et (e) étant présents dans le liant respectivement à des concentrations de 12-98,9%, 1-60%, 0,1-25%, 0-3% et 0-20% en poids par rapport au poids total dudit mélange de silicones.

15. Utilisation selon la revendication précédente, caractérisée par le fait que ladite composition et/ou lesdites silicones sont telles que définies dans l'une quelconque des revendications 2 à 12.

**Claims**

1. Cosmetic composition for the skin, in the form of an anhydrous powder mainly comprising a solid particulate phase mixed with a fatty binder containing a silicone mixture, characterized by the fact that the said silicone mixture consists of:

    (a) at least one silicone oil,
    (b) at least one silicone wax,
    (c) at least one silicone resin,
    (d) optionally at least one silicone gum, and
    (e) optionally at least one phenyl dimethicone,

    and in that the said constituents (a), (b), (c), (d) and (e) are present in the binder respectively at concentrations of 12-98.9%, 1-60%, 0.1-25%, 0-3% and 0-20% by weight relative to the total weight of the silicone mixture.

2. Composition according to the preceding claim, characterized by the fact that the concentrations of the constituents of the silicone mixture, by weight relative to the total weight of the said mixture, are the following:

    - silicone wax: 2-50%,
    - silicone resin: 0.5-15%,
    - silicone gum: 0-0.4%
    - phenyl dimethicone: 0-15%,
    - silicone oil: qs 100%.

3. Composition according to any one of the preceding claims, characterized by the fact that the said fatty binder consists of the said silicone mixture.

4. Composition according to any one of the preceding claims, characterized by the fact that the low-viscosity silicone oil is at least one linear polysiloxane consisting (except for the terminal groups) of units of formula I

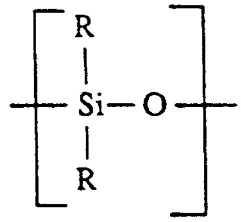

(I)

in which each substituent R independently represents a lower alkyl group having 1 to 6 C, the said polysiloxane having a degree of polymerization of 3 to 2000.

**5.** Composition according to any one of the preceding claims, characterized by the fact that the silicone wax essentially consists of at least one substituted linear polysiloxane essentially consisting (apart from the terminal groups) of units of the formulae (II) and (III), in the respective molar proportions m and n:

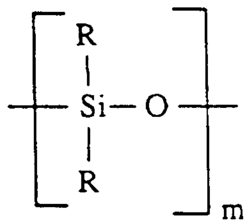 and 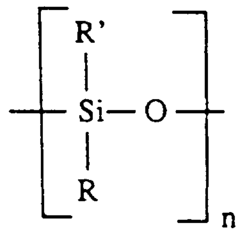

(II) (III)

in which each substituent R is an alkyl group having 1 to 6 C,
each R' independently represents an optionally unsaturated alkyl having 6 to 30 carbon atoms, or alternatively a group -X-R",
each X independently represents:
-O-,
$-(CH_2)_a-O-CO-$,
$-(CH_2)_b-CO-O-$,
a and b independently represent numbers which may range from 0 to 6, and
each R" independently represents an optionally unsaturated alkyl group having 6 to 30 carbon atoms
m is a number which may range from 0 to 400, and in particular from 0 to 100,
n is a number which may range from 1 to 200, and in particular from 1 to 100,
the sum (m + n) being less than 400, and in particular less than or equal to 100.

**6.** Composition according to any one of the preceding claims, characterized by the fact that the said silicone wax contains at least one compound of the formula IV

$$R_1-Si(CH_3)_2-O-[Si(R)_2-O-]_z-Si(CH_3)_2-R_2 \qquad (IV)$$

in which each R is an alkyl group having 1 to 6 C,
$R_1$ represents an alkyl group having 1 to 30 C, an alkoxy group having from 6 to 30 C, or a group:

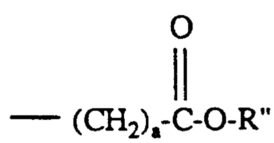

or

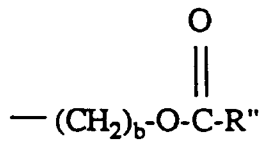

$R_2$ represents an alkyl group of 6 to 30 C, an alkoxy group having from 6 to 30 C or a group:

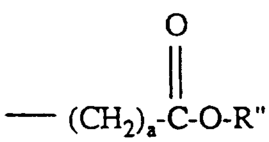

or

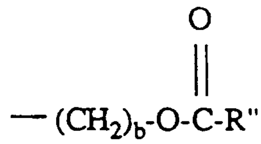

a and b representing a number from 0 to 6,
R" being a $C_6$ to $C_{30}$ alkyl,
and z is a number which may range from 1 to 100.

7. Composition according to any one of the preceding claims, characterized by the fact that the said silicone resin consists of hydrolysis and polycondensation products of mixtures of siloxanes of formula $(R)_3SiOCH_3$ and $Si(OCH_3)_4$, R representing an alkyl group having from 1 to 6 C.

8. Composition according to any one of the preceding claims, characterized by the fact that the said silicone gum is a polysiloxane having a molecular mass of 200,000 to 1 million.

9. Composition according to any one of the preceding claims, characterized by the fact that the said phenyl dimethicone corresponds to the formula VI:

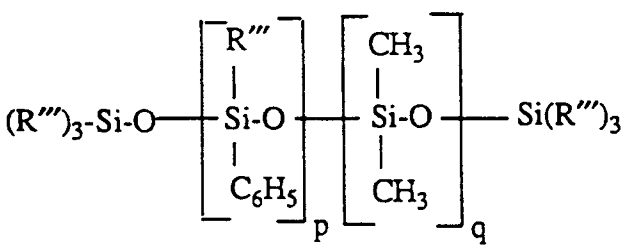

(VI)

in which
q is a number which may range from 0 to 5,000,
p is a number which may range from 1 to 5,000,
and each R''' independently represents a methyl, phenyl or trimethylsilyloxy group.

10. Composition according to any one of the preceding claims, characterized by the fact that the said silicone mixture represents from 0.5 to 25%, and in particular from 3 to 20% by weight relative to the total weight of the composition.

11. Composition according to any one of the preceding claims, characterized by the fact that it comprises, in addition, at least one additive agent chosen from antiseptics, astringent agents, sunscreen agents, cicatrizing agents, anti-free-radical agents, vitamins, demulsent agents, emollient agents, depigmenting agents, perfumes and consistency agents.

12. Composition according to the preceding claim, characterized by the fact that the additives, taken as a whole, do not represent more than 10%, and in particular not more than 5%, of the total weight of the composition.

13. Composition according to any one of the preceding claims, characterized by the fact that it is provided in the form of an eye shadow, a blusher, a face make-up powder or a body powder.

14. Use of a silicone mixture in the preparation of an anhydrous cosmetic composition provided in the form of a powder consisting of solid particles mixed with a fatty binder comprising a silicone mixture, characterized by the fact that the said silicone mixture consists of:

(a) at least one silicone oil,
(b) at least one silicone wax,
(c) at least one silicone resin,
(d) optionally at least one silicone gum, and
(e) optionally at least one phenyl dimethicone,

the said constituents (a), (b), (c), (d) and (e) being present in the binder respectively at concentrations of 12-98.9%, 1-60%, 0.1-25%, 0-3% and 0-20% by weight relative to the total weight of the said silicone mixture.

15. Use according to the preceding claim, characterized by the fact that the said composition and/or the said silicones are as defined in any one of Claims 2 to 12.

**Patentansprüche**

1. Kosmetische Zubereitung für die Haut in Form eines wasserfreien Puders, der im wesentlichen eine Phase aus mit einem fetten Bindemittel vermischten Festteilchen enthält, wobei das fette Bindemittel ein Gemisch aus Silikonen aufweist, dadurch gekennzeichnet, daß die Silikonmischung aus den folgenden Bestandteilen besteht:

(a) mindestens einem Silikonöl,
(b) mindestens einem Silikonwachs,
(c) mindestens einem Silikonharz,
(d) gegebenenfalls mindestens einem Silikongummi sowie
(e) gegebenenfalls mindestens einem Phenyldimethicon

wobei die Bestandteile (a), (b), (c), (d) sowie (e) in dem Bindemittel in einer Konzentration von 12 bis 98,9 Gew.-%, 1 bis 60 Gew.-%, 0,1 bis 25 Gew.-%, 0 bis 3 Gew.-% und 0 bis 20 Gew.-% in bezug auf das Gesamtgewicht des Silikongemisches vorhanden sind.

2. Zubereitung nach dem vorstehenden Anspruch, dadurch gekennzeichnet, daß die jeweiligen Konzentrationen der Bestandteile in Gew.-% in bezug auf das Gesamtgewicht der Mischung in dem Silikongemisch wie folgt betragen:

| | |
|---|---|
| - Silikonwachs: | 2 - 50 %, |
| - Silikonharz: | 0,5 - 15 %, |
| - Silikongummi: | 0 - 0,4 %, |
| - Phenyldimethicon: | 0 - 15 %, |
| - Silikonöl: | q.s. ad 100 %. |

3. Zubereitung nach einem der vorstehenden Ansprüche, dadurch gekennzeichnet, daß das fette Bindemittel aus dem Gemisch aus Silikonen besteht.

4. Zubereitung nach einem der vorstehenden Ansprüche, dadurch gekennzeichnet, daß das Silikonöl geringer Viskosität mindestens ein lineares Polysiloxan darstellt, das aus den folgenden Einheiten gemäß der Formel I (endständige Gruppen ausgenommen)

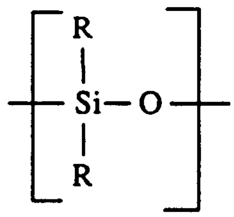

(I)

besteht, worin
der jeweilige Substituent R unabhängig einen niederen Alkylrest mit 1 bis 6 Kohlenstoffatomen darstellt, wobei das Polysiloxan einen Polymerisationsgrad von 3 bis 2000 aufweist.

**5.** Zubereitung nach einem der vorstehenden Ansprüche, dadurch gekennzeichnet, daß das Silikonwachs im wesentlichen aus mindestens einem linearen substituierten Polysiloxan besteht und sich im wesentlichen aus den folgenden Einheiten gemäß den Formeln (II) und (III) (abgesehen von den endständigen Gruppen) in den jeweiligen Molverhältnissen m und n zusammensetzt:

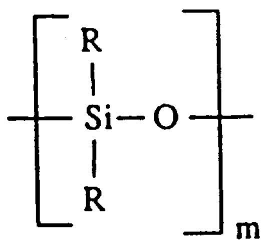

(II)

und

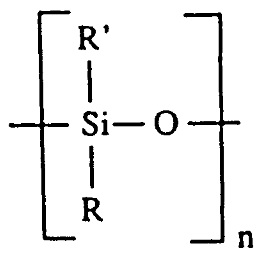

(III)

worin der jeweilige Substituent R einen Alkylrest mit 1 bis 6 Kohlenstoffatomen darstellt, und wobei jeder Rest R' unabhängig davon einen gegebenenfalls ungesättigten Alkylrest mit 6 bis 30 Kohlenstoffatomen oder auch einen Rest -XR" bedeutet, wobei X jeweils unabhängig voneinander
-O-,
$-(CH_2)_a$-O-CO-,
$-(CH_2)_b$-CO-O-,
darstellt, wobei a und b unabhängig voneinander die Zahl 0 bis 6 bedeuten können und R" unabhängig einen gegebenenfalls ungesättigten Alkylrest mit 6 bis 30 Kohlenstoffatomen bedeutet und
m eine Zahl von 0 bis 400, insbesondere von 0 bis 100 darstellen kann und n eine Zahl von 1 bis 200, insbesondere von 1 bis 100 bedeuten kann und die Summe (m + n) weniger als 400, insbesondere weniger oder gleich 100 beträgt.

**6.** Zubereitung nach einem der vorstehenden Ansprüche, dadurch gekennzeichnet, daß das Silikonwachs mindestens eine Verbindung gemäß Formel IV enthält:

$$R_1\text{-}Si(CH_3)_2\text{-}O\text{-}[Si(R)_2\text{-}O\text{-}]_z\text{-}Si(CH_3)_2\text{-}R_2 \qquad (IV)$$

worin jeder Rest R einen Alkylrest mit 1 bis 6 Kohlenstoffatomen darstellt und $R_1$ einen Alkylrest mit 1 bis 30 Kohlenstoffatomen, einen Alkoxyrest mit 6 bis 30 Kohlenstoffatomen oder die folgenden Reste bedeutet:

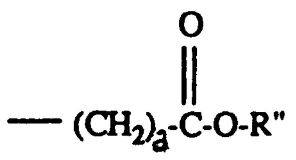

bzw.

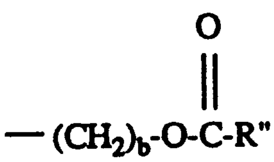

$R_2$ einen Alkylrest mit 6 bis 30 Kohlenstoffatomen, einen Alkoxyrest mit 6 bis 30 Kohlenstoffatomen oder den folgenden Rest

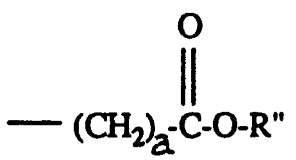

bzw.

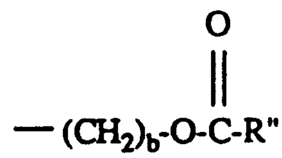

darstellt, wobei a und b eine Zahl von 0 bis 6 bedeuten,
R" einen $C_6$ bis $C_{30}$-Alkylrest bedeuten und
z eine Zahl von 1 bis 100 betragen kann.

**7.** Zubereitung nach einem der vorstehenden Ansprüche, dadurch gekennzeichnet, daß das Silikonharz aus Hydrolyseprodukten und Polykondensationsprodukten aus Siloxangemischen der Formel $(R)_3SiOCH_3$ und $Si(OCH_3)_4$ besteht, wobei R einen Alkylrest mit 1 bis 6 Kohlenstoffatomen bedeutet.

**8.** Zubereitung nach einem der vorstehenden Ansprüche, dadurch gekennzeichnet, daß der Silikongummi aus einem Polysiloxan mit einem Molekulargewicht von 200 000 bis 1 000 000 gebildet ist.

**9.** Zubereitung nach einem der vorstehenden Ansprüche, dadurch gekennzeichnet, daß das Phenyldimethicon der folgenden Formel VI entspricht:

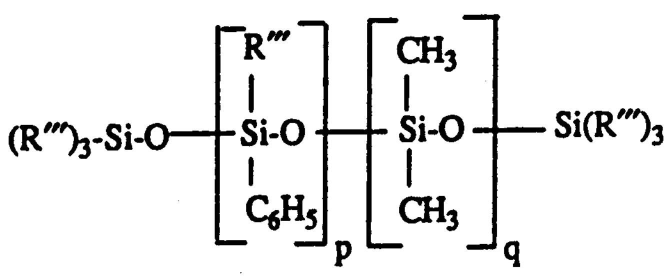

(VI)

worin
q eine Zahl von 0 bis 5000 und
p eine Zahl von 1 bis 5000 bedeuten können, wobei jeder Rest "' unabhängig eine Methyl-, Phenyl- oder Trimethylsiloxygruppe darstellt.

**10.** Zubereitung nach einem der vorstehenden Ansprüche, dadurch gekennzeichnet, daß das Silikongemisch 0,5 bis 25 Gew.-%, insbesondere 3 bis 20 Gew.-% in bezug auf das Gesamtgewicht der Zubereitung bildet.

**11.** Zubereitung nach einem der vorstehenden Ansprüche, dadurch gekennzeichnet, daß es zusätzlich noch mindestens einen Hilfsstoff enthält, der unter Antiseptika, Adstringenzien, Sonnenfiltersubstanzen, die Vernarbung fördernden Stoffen, freien antiradikalbildenden Stoffen, Vitaminen, Glättungsmitteln, erweichenden Stoffen, Pigmentierungsmittel, Duftstoffen und konsistenzgebenden Mittel ausgewählt ist.

**12.** Zubereitung nach dem vorstehenden Anspruch, dadurch gekennzeichnet, daß die Hilfsstoffe zusammengenommen nicht mehr als 10 Gew.-%, insbesondere nicht mehr als 5 Gew.-% in bezug auf das Gesamtgewicht der Zusammensetzung ausmachen.

**13.** Zubereitung nach einem der vorstehenden Ansprüche, dadurch gekennzeichnet, daß sie in Form von Augenlidschminke, Make-up, einem Puder zur Gesichtsschminke oder eines Körperpuders vorliegt.

**14.** Verwendung eines Gemisches aus Silikonen bei der Herstellung einer wasserfreien kosmetischen Zubereitung, welche in Form eines Puders vorliegt und aus mit einem fetten Bindemittel vermischten Festteilchen besteht, wobei das fette Bindemittel ein Gemisch aus Silikonen aufweist, dadurch gekennzeichnet, daß das Silikongemisch aus den folgenden Bestandteilen besteht:

(a) mindestens einem Silikonöl,
(b) mindestens einem Silikonwachs,
(c) mindestens einem Silikonharz,
(d) gegebenenfalls mindestens einem Silikongummi sowie
(e) gegebenenfalls mindestens einem Phenyldimethicon,

wobei die Bestandteile (a), (b), (c), (d) sowie (e) in dem Bindemittel in einer Konzentration von 12 bis 98,9 Gew.-%, 1 bis 60 Gew.-%, 0,1 bis 25 Gew.-%, 0 bis 3 Gew.-% und 0 bis 20 Gew.-% in bezug auf das Gesamtgewicht des Silikongemisches vorhanden sind.

**15.** Verwendung nach dem vorstehenden Anspruch, dadurch gekennzeichnet, daß die Zubereitung und/oder die Silikone die in einem der Ansprüche 2 bis 12 angegebenen Bedeutungen aufweisen.